# EUROPEAN PATENT APPLICATION

(11) **EP 1 714 618 A2**
(43) Date of publication of application: **25.10.2006**
(21) Application number: 06007302.0
(22) Date of filing: 06.04.2006
(51) Int. Cl.: A61B 17/16

(54) **Expandable reamer**

(30) Priority: 07.04.2005 US 100812
(71) Applicant: Zimmer Technology, Inc., Chicago, Illinois 60606 (US)
(72) Inventor: Krebs, Robert D., Warsaw, IN 46580 (US); Vankoski, Stephen J., Ft. Wayne, IN 46814 (US)
(74) Representative: Manitz, Finsterwald & Partner GbR

(57) **Abstract**

A bone reaming apparatus includes a rasp and a shaft coupled to the rasp. At least a first portion of the shaft is rotatable about an axis, and at least a portion of the rasp is extendable away from the axis. A method for removing a mass from a bone includes drilling a bore into the bone, and expansively reaming a portion of the bore until the mass is removed from the bone. A method for removing a femoral head from a proximal femur includes drilling a bore into the femoral head, and expansively reaming a portion of the bore until the femoral head is removed from the proximal femur.

## Description

### FIELD OF THE INVENTION

The present invention relates generally to the field of orthopaedics, and, more particularly, to a bone reaming apparatus and a method for removing a mass from a bone.

### BACKGROUND

Arthroplasty is surgery to relieve pain and restore range of motion by realigning or reconstructing a joint. Typical arthroplastic options include joint resection, interpositional reconstruction, and total joint replacement.

Joint resection involves removing a portion of a bone from a joint to create a gap between the bone and the corresponding socket, thereby improving the range of motion. Scar tissue eventually fills the gap. Pain may be relieved and motion restored, but the joint is typically less stable.

Interpositional reconstruction reshapes the joint and adds a prosthetic disk between the bones forming the joint. The prosthesis can be made of plastic and metal or from body tissue such as fascia and skin.

Joint replacement (i.e., total joint arthroplasty) is the surgical replacement of a joint with a prosthesis. If the joint does not respond to the more conservative treatments (which may include medication, weight loss, activity restriction, and/or use of walking aids such as a cane), joint replacement is often considered appropriate. Many joint replacements are needed because arthritis has caused the joint to stiffen and become painful to the point where normal daily activities are no longer possible.

Most arthroplastic procedures typically require at least some resection (i.e., removal and/or reshaping) of one or more bones of the affected joint. To provide access and working room for making the resections with traditional tools, many traditional arthroplastic procedures require lengthy incisions and substantial separations of the bones of the affected joints.

However, minimally invasive surgical techniques are becoming increasingly popular. Minimally invasive surgical techniques employ, among other things, smaller incisions, which tend to reduce tissue traumas and accelerate postoperative recoveries. But because minimally invasive techniques generally reduce the size of the surgical site, they also generally reduce the amount of space available for inserting and aligning specialized guides, saws and other resection tools.

Thus, there is an increasing need for tools that can fit through relatively small incisions and yet still remove and/or reshape substantial amounts of bone.

### SUMMARY OF THE INVENTION

The present invention provides a bone reaming apparatus. The apparatus includes a rasp and a shaft coupled to the rasp. At least a first portion of the shaft is rotatable about an axis, and at least a portion of the rasp is extendable away from the axis.

The present invention provides a method for removing a mass from a bone. The method includes drilling a bore into the bone, and expansively reaming a portion of the bore until the mass is removed from the bone.

The present invention provides a method for removing a femoral head from a proximal femur. The method includes drilling a bore into the femoral head, and expansively reaming a portion of the bore until the femoral head is removed from the proximal femur.

### BRIEF DESCRIPTION OF THE DRAWINGS

FIG. 1 shows a perspective view of a first exemplary expandable reamer according to the present invention in a first position;

FIG. 2 shows a perspective view of the exemplary expandable reamer of FIG. 1 in a second position;

FIG. 3 shows a first plan view of the exemplary expandable reamer of FIG. 1 (in the same position as FIG. 1);

FIG. 4 shows a cross-sectional view of the exemplary expandable reamer of FIG. 1 (in the same position as FIG. 1) along line 4-4 of FIG. 3;

FIG. 5 shows a second plan view of the exemplary expandable reamer of FIG. 1 (in the same position as FIG. 1);

FIG. 6 shows a cross-sectional view of the exemplary expandable reamer of FIG. 1 (in the same position as FIG. 1) along line 6-6 of FIG. 5;

FIG. 7 shows a perspective view of the cutting end of the exemplary expandable reamer of FIG. 1 in a third position;

FIG. 8 shows a cross-sectional view of the cutting end of the exemplary expandable reamer of FIG. 1 in a fourth position, along line 6-6 of FIG. 5;

FIG. 9 shows a cross-sectional view of the cutting end of the exemplary expandable reamer of FIG. 1 in a fifth position, along line 6-6 of FIG. 5;

FIG. 10 shows a cross-sectional view of the cutting end of the exemplary expandable reamer of FIG. 1 in a sixth position, along line 6-6 of FIG. 5;

FIG. 11 shows a cross-sectional view of the cutting end of the exemplary expandable reamer of FIG. 1 in a seventh position, along line 6-6 of FIG. 5;

FIG. 12 shows a perspective view of the cutting end of the exemplary expandable reamer of FIG. 1 (in the same position as FIG. 1);

FIG. 13 shows an exemplary bore through a first exemplary proximal femur that facilitates exemplary operations of the exemplary expandable reamer of FIG. 1;

FIG. 14 shows the exemplary proximal femur of FIG. 13 after its femoral head has been removed by the exemplary expandable reamer of FIG. 1;

FIG. 15 shows a second exemplary proximal femur after a generally hemispherical mass has been removed from its femoral head (without completely removing the femoral head) according to exemplary alternative operations of the present invention;

FIG. 16 shows a perspective view of an exemplary alternative expandable reamer according to the present invention;

FIG. 17 shows a perspective view of another exemplary alternative expandable reamer according to the present invention in one position; and

FIG. 18 shows a cross-sectional view of the exemplary alternative expandable reamer of FIG. 17 in another position, along line 18-18 of FIG. 17.

### DETAILED DESCRIPTION OF EXEMPLARY EMBODIMENT(S)

Like reference numerals refer to like parts throughout the following description and the accompanying drawings.

As used herein, the terms "medial," "medially," and the like mean pertaining to the middle, in or towards the middle, and/or nearer to the middle of the body when standing upright. Conversely, the terms "lateral," "laterally," and the like are used herein as opposed to medial. For example, the medial side of the knee is the side closest to the other knee and the closest sides of the knees are medially facing, whereas the lateral side of the knee is the outside of the knee and is laterally facing. Further, as used herein the term "superior" means closer to the top of the head and/or farther from the bottom of the feet when standing upright. Conversely, the term "inferior" is used herein as opposed to superior. For example, the heart is superior to the stomach and the superior surface of the tongue rests against the palate, whereas the stomach is inferior to the heart and the palate faces inferiorly towards the tongue. Additionally, as used herein the terms "anterior," "anteriorly," and the like mean nearer the front or facing away from the front of the body when standing upright, as opposed to "posterior," "posteriorly," and the like, which mean nearer the back or facing away from the back of the body.

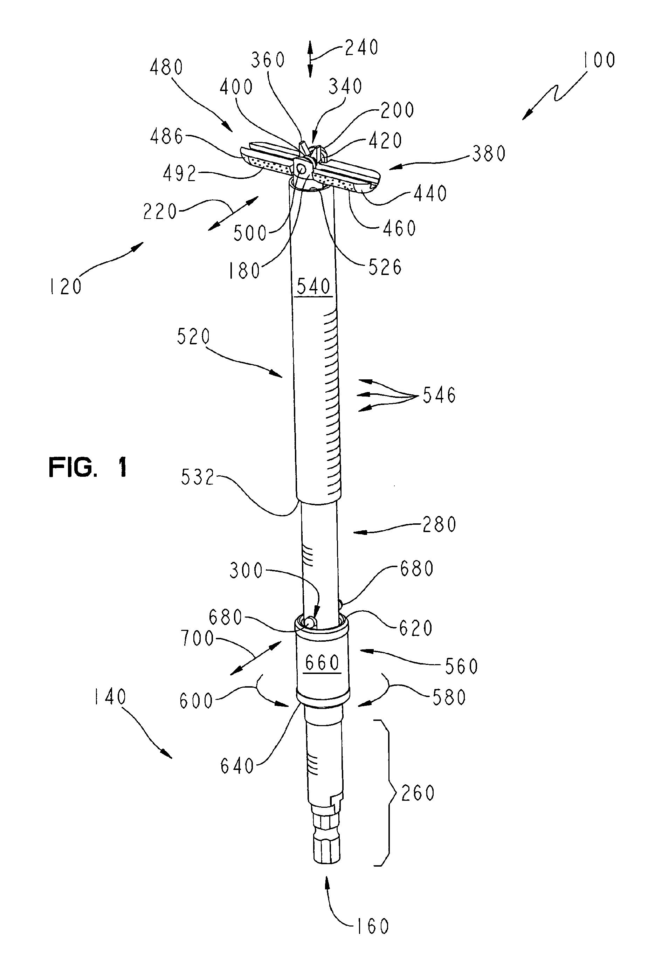

FIG. 1 shows a perspective view of a first exemplary expandable reamer 100 according to the present invention in a first position. In the exemplary embodiment, reamer 100 is an elongated, snake-like device with a cutting end 120 and a drive-coupling end 140. As discussed further below, reamer 100 is configured to be inserted into a bore within a bone in a retracted position and to expansively ream out a portion of the bore after insertion.

Exemplary reamer 100 includes a hollow shaft 160. Among other things, shaft 160 is configured to transmit torque from a suitable rotary power source (not shown) from end 140 to end 120 of reamer 100, and shaft 160 is configured to cooperate in extending and retracting reamer 100.

At end 120, shaft 160 includes an eyelet 180 and an opposing, identically configured eyelet 200. In the exemplary embodiment, eyelet 180 and eyelet 200 are made from stainless steel. In alternative embodiments, eyelet 180 and eyelet 200 may be made from any other suitable material(s). Eyelet 180 and eyelet 200 are both centered about an axis 220. Among other things, shaft 160 is configured to transmit rotational force from the rotary power source to rotate eyelet 180 and eyelet 200 (and, thus, axis 220 as well) about an axis 240. Axis 240 is perpendicular to axis 220, and eyelet 180 and eyelet 200 are both equidistant from axis 240.

At end 140, shaft 160 includes a male hexagonal quick-disconnect stud 260. Stud 260 is configured to couple reamer 100 to a suitable rotary power source. In alternative embodiments, shaft 160 may define any other suitable male and/or female member or assembly for coupling reamer 100 to a suitable rotary power source. In the exemplary embodiment, stud 260 is made from stainless steel. In alternative embodiments, stud 260 may be made from any other suitable material(s).

Extending between eyelet 180 (and eyelet 200) and stud 260, shaft 160 also includes a tubular portion 280. In the exemplary embodiment, portion 280 is straight when relaxed but also flexible yet resilient and/or elastic (such that it is deformable in response to the application of a force yet automatically resumes its original shape upon removal of the force), and is made from a shaped memory alloy (i.e., a memory metal that deforms in response to a force but automatically resumes its original shape upon removal of the force). Such shaped memory alloys may include an alloy of nickel and titanium such as Nitinol manufactured by the Nitinol Devices and Components, Fremont CA. In alternative embodiments, portion 280 may be curved, flexible yet resilient and/or elastic, or flexible but neither resilient nor elastic (such that it is deformable in response to the application of a force and does not automatically resume its original shape upon removal of the force), and may be made from any other suitable material(s). Portion 280 defines an oblong slot 300 (only partially visible in FIG. 1) and an opposing, identically configured oblong slot 320 (not visible in FIG. 1). Portion 280 is discussed further below.

Next, reamer 100 further includes a shaft 340 coaxially extending into shaft 160 along axis 240. Shaft 340 is axially slidable within shaft 160. Among other things, shaft 340 is configured to cooperate in extending and retracting reamer 100. Shaft 340 includes a notched end portion 360 that protrudes from shaft 160 at end 120. In the exemplary embodiment, portion 360 is made from stainless steel. In alternative embodiments, portion 360 may be made from any other suitable material(s). Shaft 340 is discussed further below.

Reamer 100 further includes a rasp 380. Among other things, rasp 380 is configured to rasp bone. In the exemplary embodiment, rasp 380 is made from stainless steel. In alternative embodiments, rasp 380 may be made from any other suitable material(s). Rasp 380 includes a smooth eyelet 400 and an opposing smooth eyelet 420. Eyelet 400 and eyelet 420 are coaxially centered. Rasp 380 is positioned such that eyelet 400 and eyelet 420 are concentrically aligned along axis 220. Rasp 380 also includes a curved, blunt end surface 440 and a curved, serrated surface 460 extending between eyelet 400, eyelet 420 and surface 440. Rasp 380 is discussed further below.

Reamer 100 further includes a rasp 480. Rasp 480 is configured identically to rasp 380. Accordingly, rasp 480 includes a curved, blunt end surface 486 and a curved, serrated surface 492, among other things. Rasp 480 is discussed further below.

Reamer 100 further includes a smooth hinge pin 500. Pin 500 is configured to pivotally couple rasp 380 and rasp 480 to shaft 160. In the exemplary embodiment, pin 500 is made from stainless steel. In alternative embodiments, pin 500 may be made from any other suitable material(s). Pin 500 is inserted, along axis 220, through the concentrically aligned eyelets of shaft 160, rasp 380, and rasp 480. In the exemplary embodiment, pin 500 is welded to eyelet 180 and eyelet 200 of shaft 160. In alternative embodiments, pin 500 may be secured within eyelet 180 and/or eyelet 200 in any other suitable manner. Rasp 380 and rasp 480 (via their respective eyelets) are left free to pivot (about axis 220) on pin 500.

Reamer 100 further includes a tubular sleeve 520 slidably fitted around portion 280. Sleeve 520 includes an annular surface or rim 526, an annular surface or rim 532, and an outer sidewall surface 540 extending between rim 526 and rim 532. Among other things, sleeve 520 is configured to cover the serrated portions of rasp 380 and rasp 480 during insertion of reamer 100 into a bore as discussed further below. In the exemplary embodiment, sleeve 520 is made from stainless steel. In alternative embodiments, sleeve 520 may be made from any other suitable material(s). Along surface 540, reamer 100 has a plurality of markings or graduations 546.

Reamer 100 also includes an actuation collar 560. Collar 560 is screw-threaded or otherwise spirally coupled around portion 280 such that rotation of collar 560 in a direction 580 relative to portion 280 forces axial translation of collar 560 towards end 120 of reamer 100, and, conversely, such that counter-rotation of collar 560 in a direction 600 relative to portion 280 forces axial translation of collar 560 towards end 140 of reamer 100. Collar 560 includes an annular surface or rim 620 proximal to end 120, an annular surface or rim 640 proximal to end 140, and a tubular sidewall 660 extending between rim 620 and rim 640.

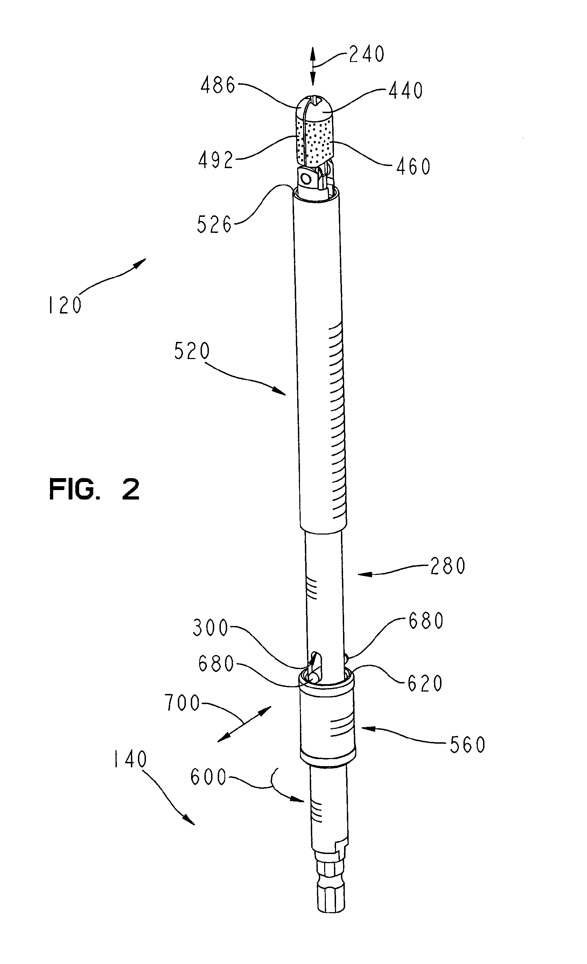

Reamer 100 also includes a coupling pin 680. Pin 680 extends- along an axis 700 - through slot 300, shaft 340, and slot 320. Further, pin 680 is positioned such that when reamer 100 is straightened, axis 700 is parallel to axis 220, perpendicular to axis 240, and closer to end 120 than rim 620 of collar 560. Pin 680 is slidable within slot 300 and slot 320 such axis 700 is translatable along shaft 160. It should be appreciated that in FIG. 1 (compare FIG. 2), collar 560 has been rotated around portion 280 (along direction 580) until rim 620 has forced pin 680 to stop in slot 300 (and slot 320), such that axis 700 is proximal to end 120 of reamer 100 (and distal to end 140).

FIG. 2 shows a perspective view of reamer 100 in a second position. Among other things, axis 240, portion 280, slot 300, surface 440, surface 460, surface 486, surface 492, sleeve 520, rim 526, collar 560, rim 620, and pin 680 are all at least partially discernable in FIG. 2. It should be appreciated that in FIG. 2 (compare FIG. 1), collar 560 has been rotated around portion 280 (along direction 600) such that rim 620 has translated towards end 140 of reamer 100 (and away from end 120), thereby freeing pin 680 to move in slot 300 (and slot 320; not visible in FIG. 2) such that axis 700 is free to translate towards end 140 of reamer 100 (and away from end 120).

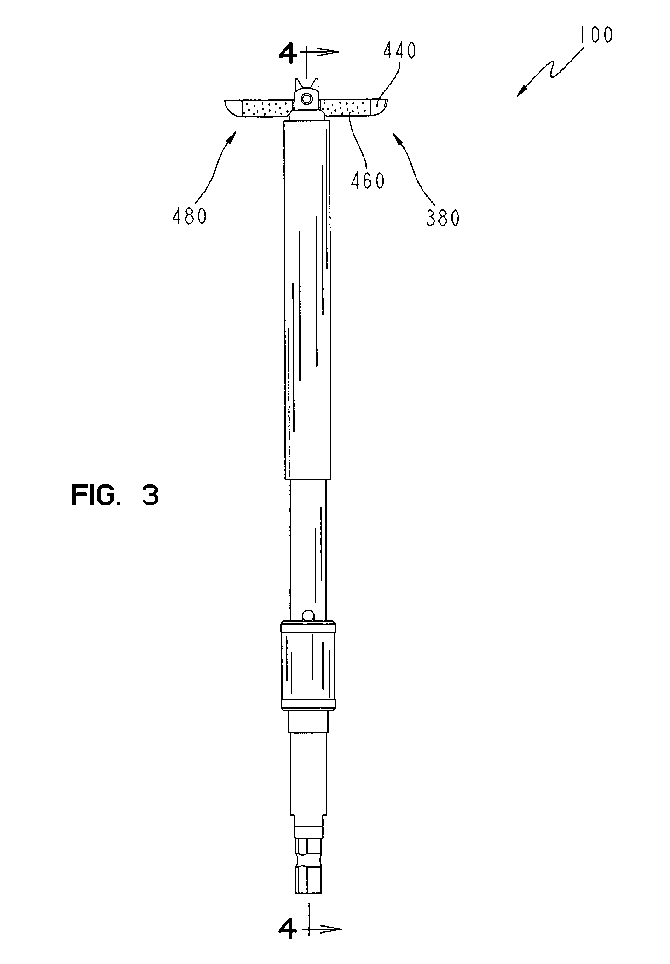

FIG. 3 shows a first plan view of reamer 100 (in the same position as FIG. 1). Rasp 380, surface 440 and surface 460 of rasp 380, and rasp 480, among other things, are all at least partially discernable in FIG. 3.

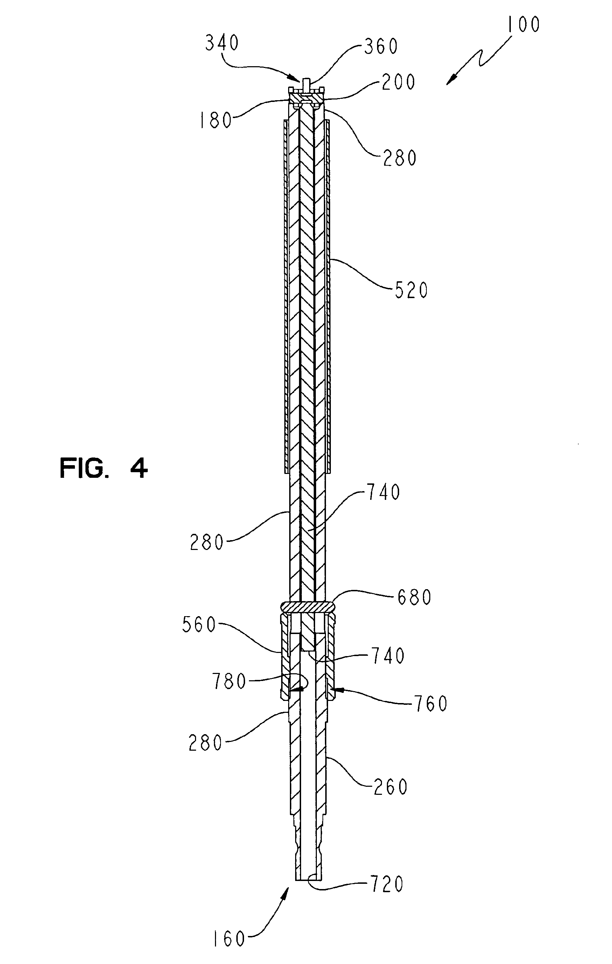

FIG. 4 shows a cross-sectional view of reamer 100 (in the same position as FIG. 1) along line 4-4 of FIG. 3. As discernable in FIG. 4, shaft 160 includes a cylindrical bore 720. Bore 720 extends coaxially throughout shaft 160. Further, shaft 340 includes a smooth cylindrical portion 740 that extends from portion 360 into bore 720. Further, portion 280 of shaft 160 defines screw threads 760 and collar 560 defines matching screw threads 780. Threads 760 and threads 780 spirally couple collar 560 to portion 280 of shaft 160 as discussed above. Additionally, eyelet 180, eyelet 200, portion 260 of shaft 160, portion 280 of shaft 160, sleeve 520, collar 560, and pin 680, among other things, are all at least partially discernable in FIG. 4.

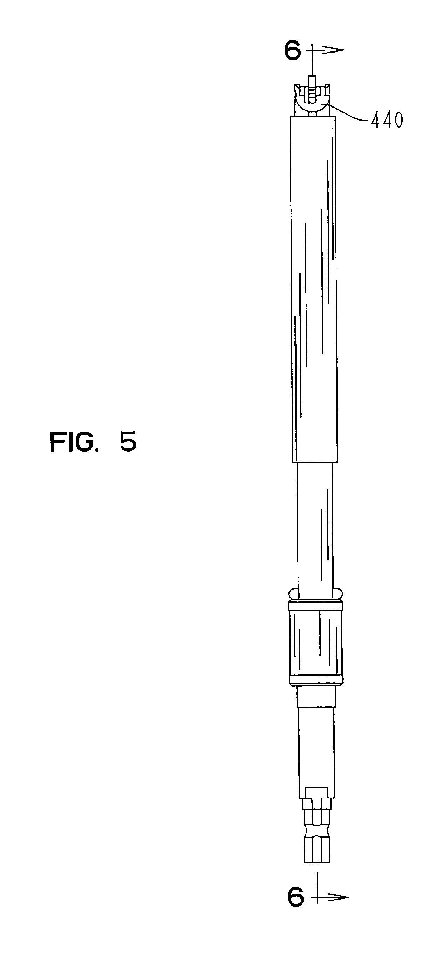

FIG. 5 shows a second plan view of reamer 100 (in the same position as FIG. 1). Surface 440 (of rasp 380; see FIG. 1 and FIG. 3), among other things, is at least partially discernable in FIG. 5.

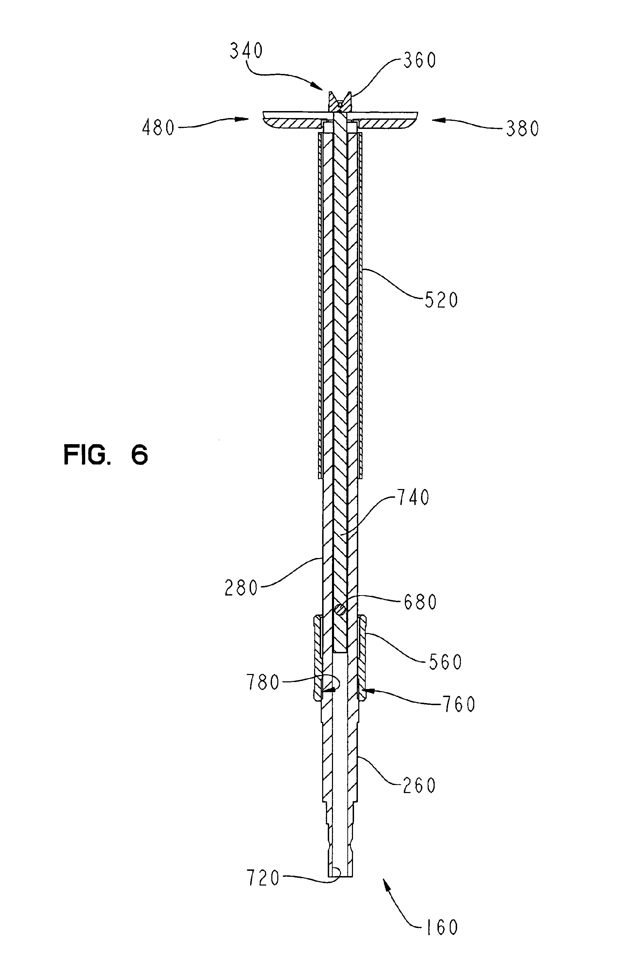

FIG. 6 shows a cross-sectional view of reamer 100 (in the same position as FIG. 1) along line 6-6 of FIG. 5. Portion 260 of shaft 160, portion 280 of shaft 160, portion 360 of shaft 340, rasp 380, rasp 480, sleeve 520, collar 560, pin 680, bore 720, portion 740 of shaft 340, threads 760, and threads 780, among other things, are all at least partially discernable in FIG. 6.

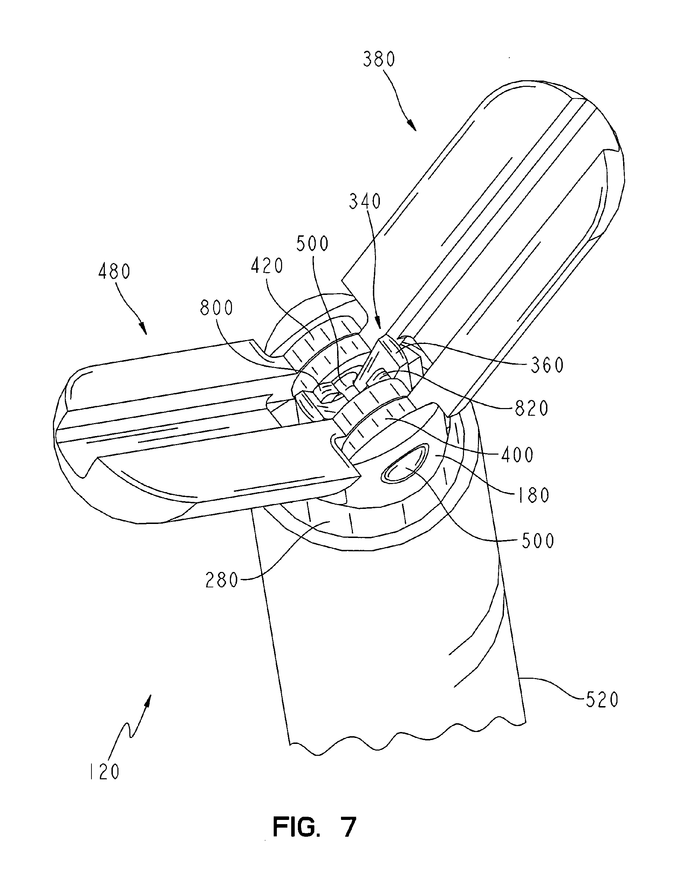

FIG. 7 shows a perspective view of end 120 of reamer 100 in a third position. As discernable in FIG. 7, rasp 380 includes a tab-like protuberance 800 extending inward from eyelet 420 towards eyelet 400. As noted above, rasp 480 is configured identically to rasp 380. Accordingly, rasp 480 includes a like protuberance 820. Additionally, eyelet 180 of shaft 160, portion 280 of shaft 160, portion 360 of shaft 340, sleeve 520, and pin 500, among other things, are all at least partially discernable in FIG. 7.

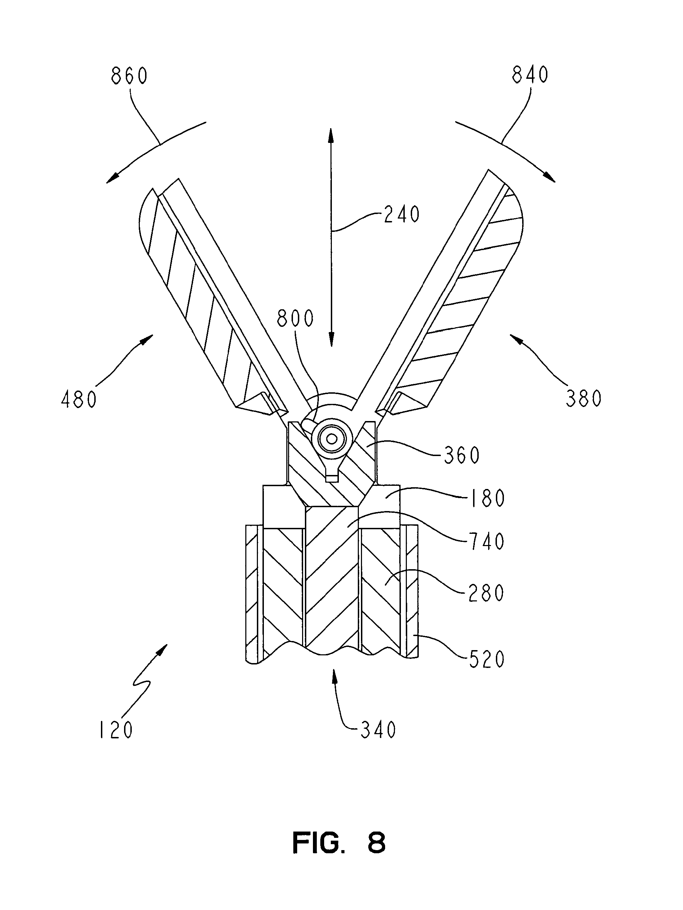

FIG. 8 shows a cross-sectional view of end 120 of reamer 100 in a fourth position, along line 6-6 of FIG. 5. To force rasp 380 to pivot radially outward from axis 240 (as indicated by directional line 840), collar 560 (see FIG. 1, FIG. 3, FIG. 4, FIG. 5, and FIG. 6) is rotated relative to portion 280 to force axial translation of collar 560 towards end 120. The translation of collar 560 towards end 120 forces pin 680 (see FIG. 1 and FIG. 4) to translate in slot 300 and slot 320 (see FIG. 1 and FIG. 4) towards end 120, which, in turn, forces portion 740 of shaft 340 to extend from portion 280 of shaft 160 (see also FIG. 1) towards protuberance 800 of rasp 380. The extension of portion 740 towards protuberance 800 forces portion 360 of shaft 340 against protuberance 800, which, in turn, forces protuberance 800 to pivot. As protuberance 800 is part of rasp 380, the pivoting of protuberance 800 forces rasp 380 to pivot radially outward from axis 240 (as indicated by directional line 840). It should be appreciated that rasp 480 is similarly simultaneously forced to pivot radially outward from axis 240 (as indicated by directional line 860) as portion 360 of shaft 340 is forced against protuberance 820 of rasp 480 (see, e.g., FIG. 7). At least partially discernable in FIG. 8 are eyelet 180 and sleeve 520, among other things.

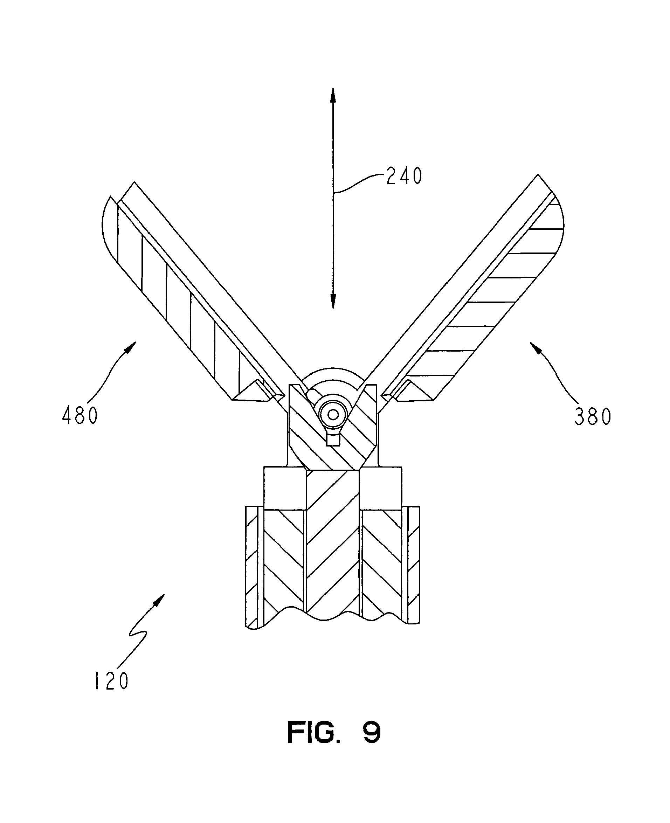

FIG. 9 shows a cross-sectional view of end 120 of reamer 100 in a fifth position, along line 6-6 of FIG. 5. In FIG. 9, reamer 100 is somewhat more extended than it is in FIG. 8. That is, rasp 380 and rasp 480 are pivoted radially outward from axis 240 more in FIG. 9 than in FIG. 8.

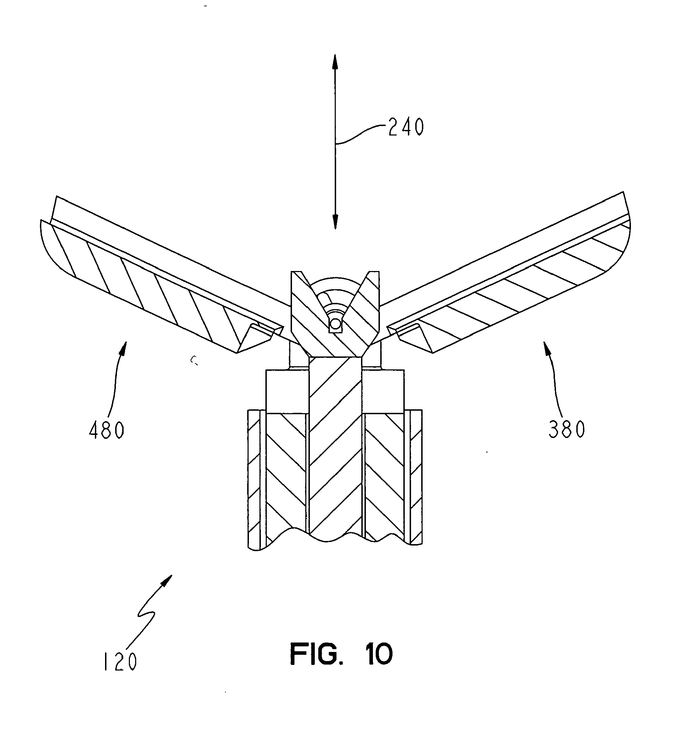

FIG. 10 shows a cross-sectional view of end 120 of reamer 100 in a sixth position, along line 6-6 of FIG. 5. In FIG. 10, reamer 100 is somewhat more extended than it is in FIG. 9. That is, rasp 380 and rasp 480 are pivoted radially outward from axis 240 more in FIG. 10 than in FIG. 9.

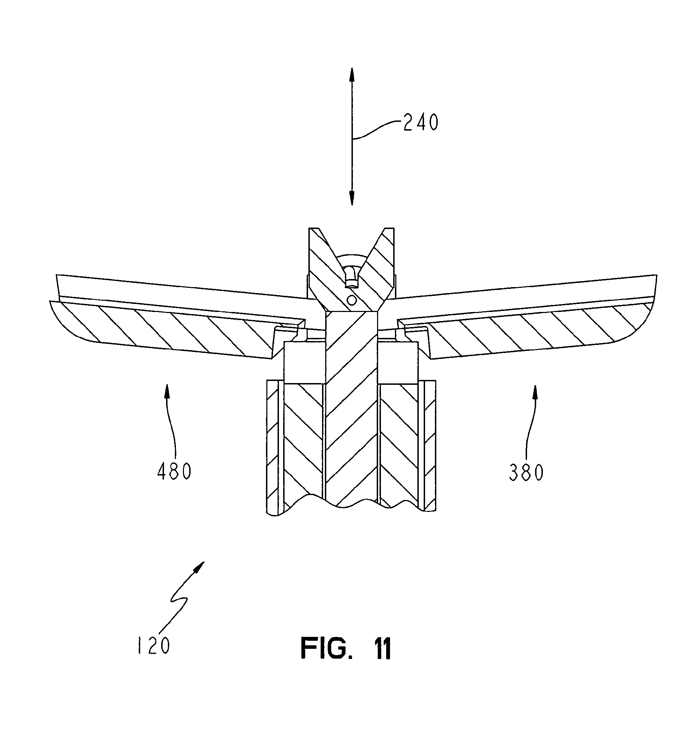

FIG. 11 shows a cross-sectional view of end 120 of reamer 100 in a seventh position, along line 6-6 of FIG. 5. In FIG. 11, reamer 100 is somewhat more extended than it is in FIG. 10. That is, rasp 380 and rasp 480 are pivoted radially outward from axis 240 more in FIG. 11 than in FIG. 10.

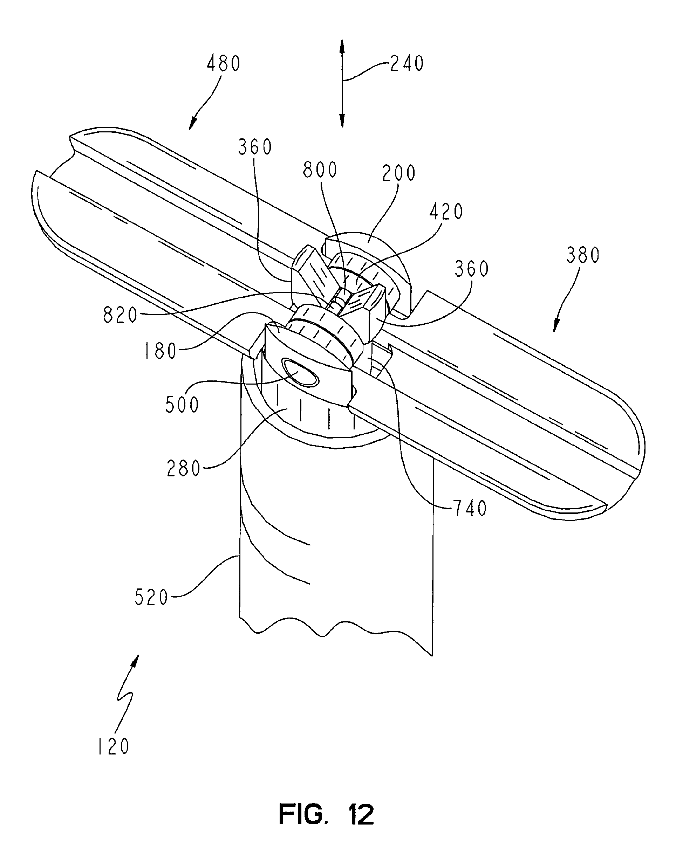

FIG. 12 shows a perspective view of end 120 of reamer 100 (in the same position as FIG. 1). In FIG. 12, reamer 100 is fully extended. That is, rasp 380 and rasp 480 are fully pivoted radially outward from axis 240. Additionally, eyelet 180, eyelet 200, portion 280, portion 360, eyelet 420, pin 500, sleeve 520, portion 740, protuberance 800, and protuberance 820, among other things, are all at least partially discernable in FIG. 12.

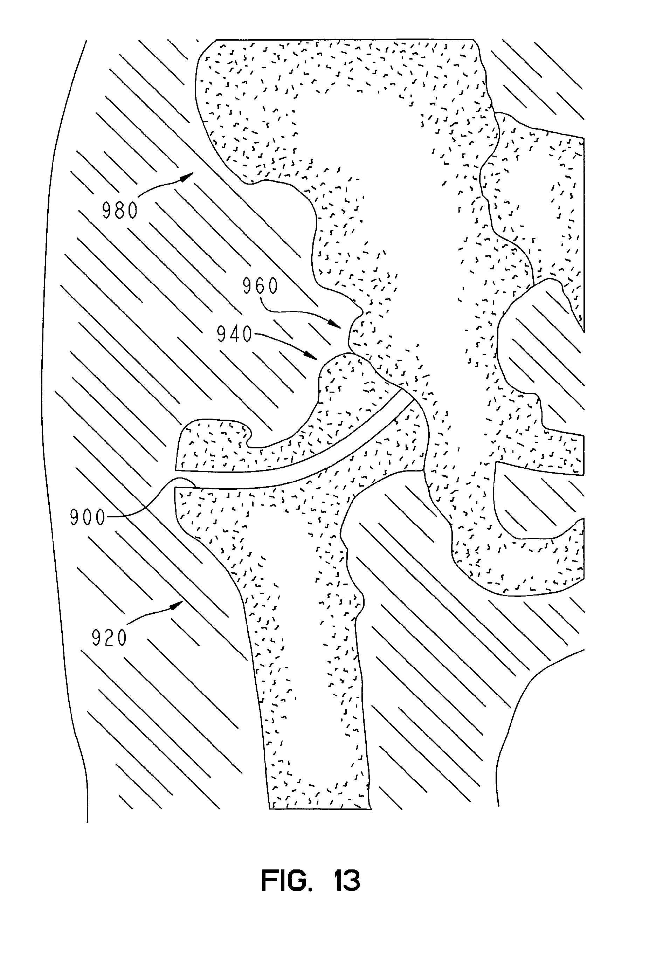

FIG. 13 shows an exemplary bore 900 through a first exemplary proximal femur 920 that facilitates exemplary operations of reamer 100. Proximal femur 920 includes a typical end portion or femoral head 940 that extends into a typical socket portion or acetabulum 960 of a hip bone 980. A suitable surgical incision is made and bore 900 is suitably pre-drilled to facilitate removal of femoral head 940 using reamer 100 as discussed further below.

To remove femoral head 940 from proximal femur 920 using reamer 100, a user rotates collar 560 around portion 280 (along direction 600) such that rim 620 translates towards end 140. Then, the user pivotally retracts rasp 380 and rasp 480 towards axis 240 (see FIG. 2). Additionally, the user slides sleeve 520 towards end 120 such that sleeve 520 covers surface 460 of rasp 380 and surface 492 of rasp 480. Further, the user couples a suitable rotary power source to portion 260 of shaft 160. Next, the user inserts end 120 of reamer 100 into bore 900 until portion 440 of rasp 380 (and portion 486 of rasp 480) reach acetabulum 960. Next, the user slides sleeve 520 back towards end 140 of reamer 100 to uncover surface 460 and surface 492 (see FIG. 2). Then, the user applies power from the rotary power source to rotate shaft 160 (and, thus, rasp 380 and rasp 480) about axis 240 in the direction of line 600 (see FIG. 1 and FIG. 2). Centrifugal forces generated by the rotation cause rasp 380 and rasp 480 to pivotally extend away from axis 240. Additionally, the user manually forces rasp 380 and rasp 480 to further pivotally extend away from axis 240 by rotating collar 560 relative to portion 280 (along direction 580). The user may cause such relative rotation of collar 560 by gripping collar 560 while shaft 160 rotates in the direction of line 600, which in turn forces rasp 380 and rasp 480 to further pivotally extend away from axis 240 without interrupting the rotation of shaft 160. Alternatively, the user may alternatively turn off the rotary power source, rotate collar 560 relative to portion 280 (to incrementally pivotally extend rasp 380 and rasp 480 away from axis 240), and re-apply the rotary power. In any event, it should be appreciated that pivotal extension of rasp 380 away from axis 240 also results in radial extension of surface 440 (among other portions of rasp 380) away from axis 240 and that, similarly, pivotal extension of rasp 480 away from axis 240 also results in radial extension of surface 486 (among other portions of rasp 480) away from axis 240. As rasp 380 and rasp 480 pivotally extend away from axis 240, surface 460 and surface 492 expansively ream out a medial portion of bore 900 until reamer 100 removes femoral head 940 from proximal femur 920. Additionally, the user may manipulate reamer 100 in a general medial to lateral motion and/or vice versa with reference to graduations 546 to gage the insertion depth or position of end 120.

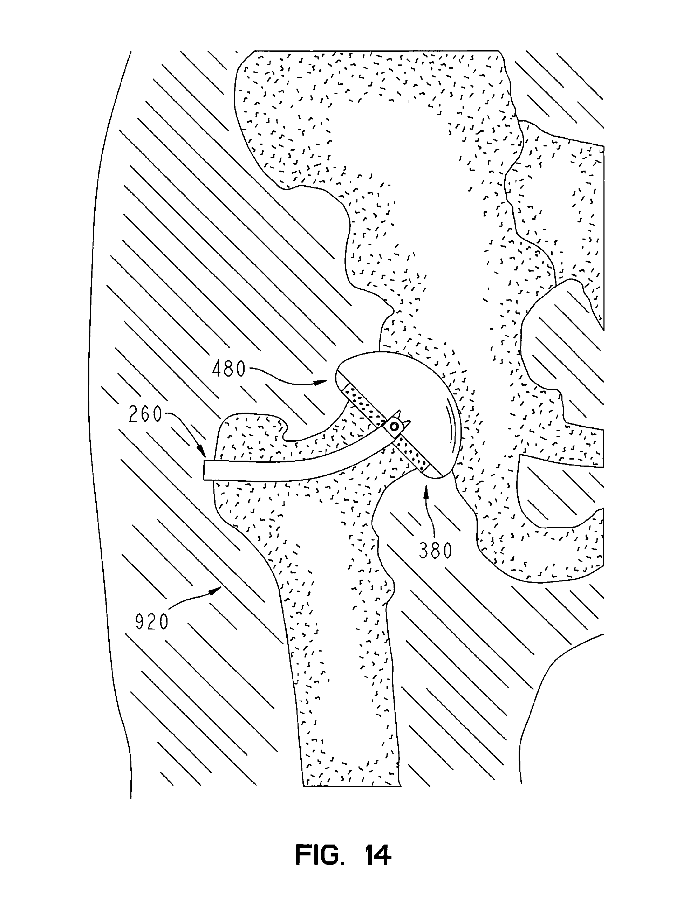

FIG. 14 shows proximal femur 920 after its femoral head 940 (see FIG. 13) has been removed by reamer 100. Portion 260, rasp 380, and rasp 480 of reamer 100, among other things, are all at least partially discernable in FIG. 14.

After reamer 100 has removed femoral head 940 (compare FIG. 13 and FIG. 14), the user turns off the rotary power source, rotates collar 560 around portion 280 (along direction 600) such that rim 620 translates towards end 140, pivotally retracts rasp 380 and rasp 480 towards axis 240 (see FIG. 2), slides sleeve 520 towards end 120 such that sleeve 520 covers surface 460 of rasp 380 and surface 492 of rasp 480, and withdraws reamer 100 from bore 900.

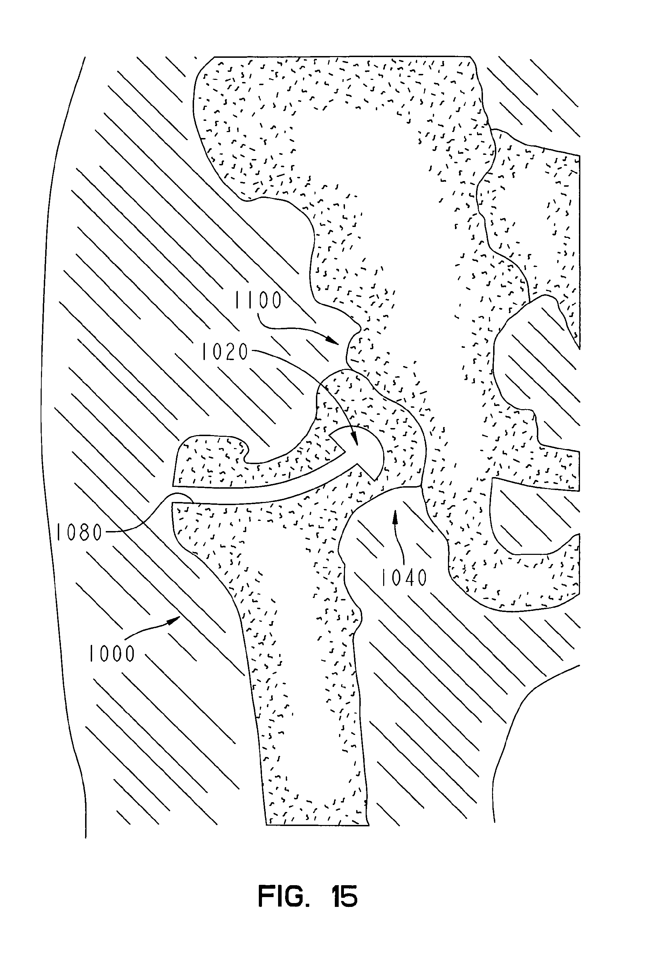

FIG. 15 shows a second exemplary proximal femur 1000 after a generally hemispherical mass (indicated by the resulting cavity 1020) has been removed from its femoral head 1040 (without completely removing the femoral head 1040) by an alternative expandable reamer 1060 (not shown) according to the present invention. Reamer 1060 is identically configured to reamer 100 with the exception that its two rasps are shorter than rasp 380 and rasp 480 such that their smooth portions do not extend as far radially away from their axis of rotation. A user operates reamer 1060 in a like manner to reamer 100 as discussed above, with the exception that pre-drilled bore 1080 does not extend all the way through femoral head 1040 to the acetabulum 1100.

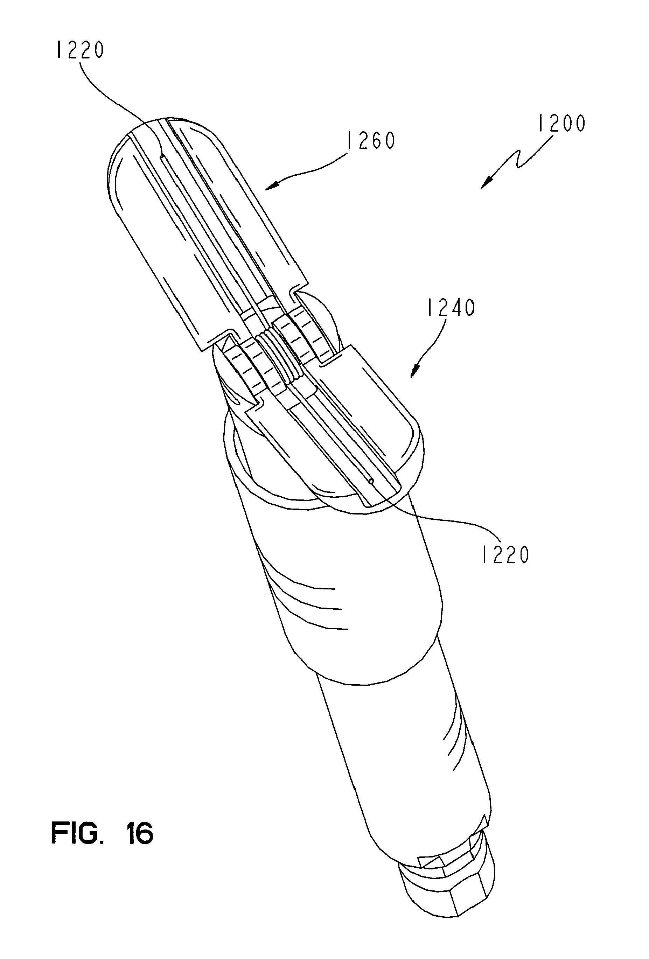

FIG. 16 shows a perspective view of an exemplary alternative expandable reamer 1200 according to the present invention. Reamer 1200 is configured and operated in a like manner to reamer 100 (discussed above), with the exceptions that shaft 340, collar 560, pin 680, protuberance 800, and protuberance 820 are omitted from reamer 1200, and, instead, a torsion spring 1220 is added to help pivot an alternative rasp 1240 and an identically configured opposing alternative rasp 1260.

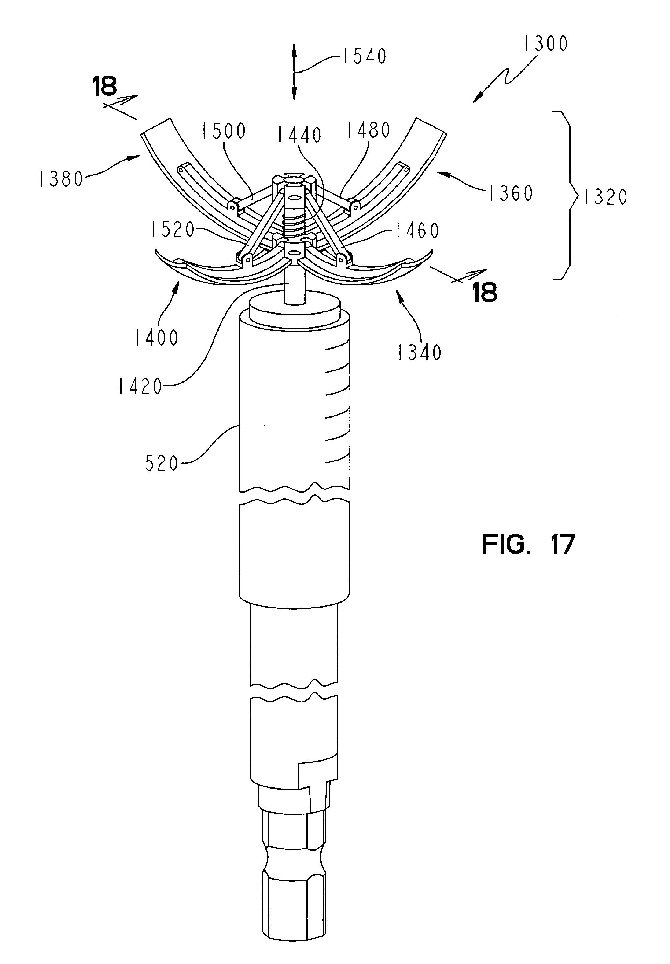

FIG. 17 shows a perspective view of another exemplary alternative expandable reamer 1300 according to the present invention in one position. Reamer 1300 is configured and operated in a like manner to reamer 1200 (discussed above), with the exceptions that eyelet 180, eyelet 200, pin 500, spring 1220, rasp 1240, and rasp 1260 are omitted from reamer 1300, and, instead, an inverted umbrella-like rasp assembly 1320 which includes an alternative rasp 1340, an identical alternative rasp 1360, an identical alternative rasp 1380, and an identical alternative rasp 1400 is added. Assembly 1320 includes a post 1420, a tension spring 1440, a link 1460, a link 1480, a link 1500, and a link 1520 to help pivot rasp 1340, rasp 1360, rasp 1380, and rasp 1400 radially outward from axis 1540 (which is the axis about which they rotate during operation). Sleeve 520, among other things, is also at please partially discernable in FIG. 17.

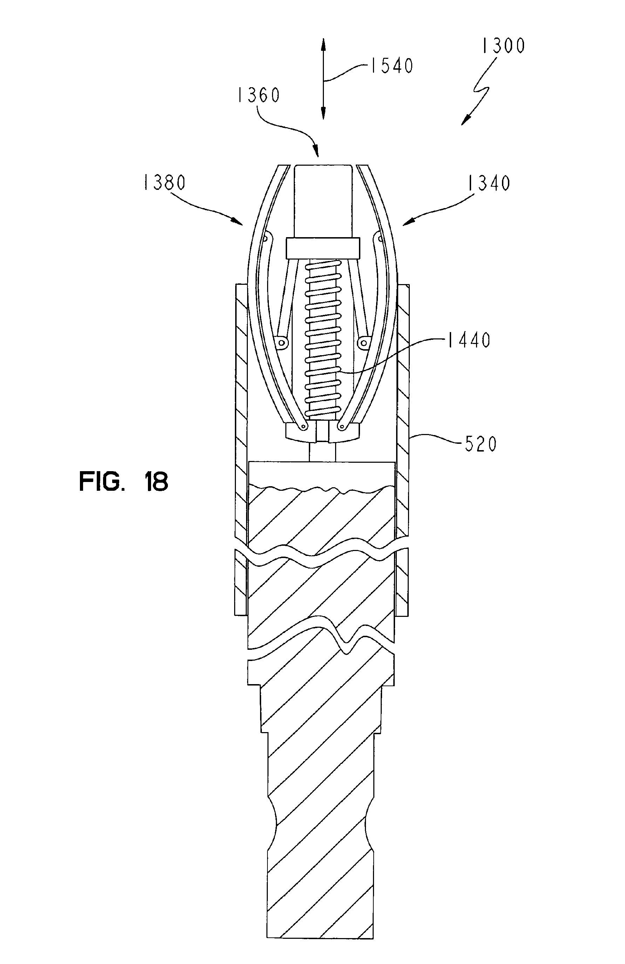

FIG. 18 shows a cross-sectional view of expandable reamer 1300 in another position, along line 18-18 of FIG. 17. In FIG. 18, sleeve 520 has been slid away from end 140 to partially cover rasp 1340, rasp 1360, rasp 1380, and rasp 1400 (which is not visible in FIG. 18; but see FIG. 17) such that they are pivotally retracted towards axis 1540 and such that the tension of spring 1440 is increased relative to the position shown in FIG. 17. It should be appreciated that sliding sleeve 520 back towards end 140 allows spring 1440, link 1460, link 1480, link 1500, and link 1520 to help pivot rasp 1340, rasp 1360, rasp 1380, and rasp 1400 radially outward from axis 1540.

It is noted that in other alternative embodiments of the present invention, an axial bore (not shown) may extend all the way through shaft 340 and/or other comparable parts. Such embodiments allow for the application of suction through shaft 160 (via bore 720) and shaft 340, which may facilitate removal of bone debris, blood, and/or other suitable surgical site waste.

Additionally, it is noted that alternative embodiments of the present invention may include a clutch mechanism or assembly (not shown) interposed between portion 280 of shaft 160 and collar 560 (see FIG. 1) such that gripping collar 560 while shaft 160 rotates will freely torque collar 560 relative to portion 280 until translation of pin 680 is stopped by slot 300 (and slot 320) and thereafter allow collar 560 to slip around portion 280 (i.e., torque limit collar 560 relative to portion 280). Such a clutch limits transmission of torque from the rotary power source through collar 560 to a user's fingers when the user grips collar 560 while shaft 160 rotates.

The foregoing description of the invention is illustrative only, and is not intended to limit the scope of the invention to the precise terms set forth. Further, although the invention has been described in detail with reference to certain illustrative embodiments, variations and modifications exist within the scope and spirit of the invention as described and defined in the following claims.

The invention can be used in a method for removing a mass from a bone, the method comprising: drilling a bore into the bone; and expansively reaming a portion of the bore until the mass is removed from the bone.
Preferably, the expansively reaming includes removing the mass from a femoral head. Preferably, the drilling step includes extending the bore completely through the bone. Preferably, the expansively reaming includes expansively reaming a portion of the bore until a generally hemispherical mass is removed from the bone. Preferably, the expansively reaming includes removing the generally hemispherical mass from a femoral head without completely removing the femoral head. The invention can be used in a method for removing a femoral head from a proximal femur, the method comprising: drilling a bore into the femoral head; and expansively reaming a portion of the bore until the femoral head is removed from the proximal femur.

## Claims

1. Bone reaming apparatus, comprising:
a means (380, 480; 1240, 1260; 1340, 1360, 1380, 1400) for cutting the bone;
a means (160), coupled to the cutting means, for rotating the cutting means about an axis (240); and
a means (340, 560) for extending at least a portion of the cutting means away from the axis (240).

2. Apparatus according to claim 1,
**characterized in that**
the extending means (340, 560) includes a means (680, 740) for extending the portion of the cutting means away from the first axis (240) while the cutting means rotates about the axis (240).

3. Apparatus according to claim 1 or 2,
**characterized in that**
the cutting means is pivotally coupled to the rotating means (160).

4. Apparatus according to any of the preceding claims,
**characterized in that**
the apparatus further includes a means (340, 560) for retracting the portion of the cutting means towards the axis (240).

5. Apparatus according to any of the preceding claims,
**characterized in that**
the cutting means is a rasp (340, 480; 1240, 1260; 1340, 1360, 1380, 1400); and
the rotating means is a shaft (160) coupled to the rasp;
wherein at least a first portion of the shaft (160) is rotatable about an axis (240), and at least a portion of the rasp is extendable away from the axis (240).

6. Apparatus according to claim 5,
**characterized in that**
the rasp is retractably extendable between a minimum radial extension from the axis (240) and a maximum radial extension from the axis (240).

7. Apparatus according to claim 6,
**characterized in that**
the rasp is continuously extendable between the minimum radial extension and the maximum radial extension.

8. Apparatus according to any of claims 5 to 7,
**characterized in that**
the portion of the rasp is extendable away from the axis (240) while the first portion of the shaft (160) rotates about the axis (240).

9. Apparatus according to any of claims 5 to 8,
**characterized in that**
the apparatus further comprises:
a sleeve or tubular portion (280, 520) covering at least a portion of the shaft (340, 280);
wherein the shaft (340, 280) is axially movable within the sleeve or tubular portion (280, 520) to cause extension of the portion of the rasp away from the axis (240).

10. Apparatus according to claim 9,
**characterized in that**
the shaft (340, 280) is axially movable within the sleeve or tubular portion (280, 520) to permit retraction of the portion of the rasp towards the axis (240).

11. Apparatus according to any of claims 5 to 10,
**characterized in that**
the apparatus further comprises:
a sleeve or tubular portion (520, 280) covering at least a portion of the shaft (280, 340);
wherein the shaft (280, 340) is axially movable within the sleeve or tubular portion (520, 280) to permit extension of the portion of the rasp away from the axis (240).

12. Apparatus according to claim 11,
**characterized in that**
the shaft (280, 340) is axially movable within the sleeve or tubular portion (520, 280) to cause retraction of the portion of the rasp towards the axis (240).

13. Apparatus according to claim 10 or 12,
**characterized in that**
the apparatus further comprises:
a screw-threaded member (560) engaged with the sleeve or tubular portion (280, 520);
wherein the screw-threaded member (560) is movable relative to the sleeve or tubular portion (280, 520) to cause axial movement of the shaft (340, 280) within the sleeve or tubular portion (280, 520).

14. Apparatus according to any of claims 5 to 13,
**characterized in that**
the shaft (160) includes a bendable portion (280).

15. Apparatus according to claim 14,
**characterized in that**
the bendable portion (280) includes an elastic portion.

16. Apparatus according to any of claims 5 to 15,
**characterized in that**
the portion of the rasp is arcuately extendable away from the axis (240).

17. Apparatus according to any of claims 5 to 16,
**characterized in that**
the rasp is pivotally coupled to the shaft (160).
